# EUROPEAN PATENT SPECIFICATION

(11) **EP 3 989 045 B1**
(45) Date of publication and mention of the grant of the patent: **29.07.2026**
(21) Application number: 21203611.5
(22) Date of filing: 20.10.2021
(51) Int. Cl.: G06F 3/01, A61B 5/16, A61B 5/00, A61B 5/11, B60K 35/10

(54) **SYSTEM AND METHOD FOR DETERMINING AN EYE MOVEMENT**
SYSTEM UND VERFAHREN ZUR BESTIMMUNG EINER AUGENBEWEGUNG
SYSTÈME ET PROCÉDÉ POUR DÉTERMINER UN MOUVEMENT OCULAIRE

(30) Priority: 20.10.2020 WO PCT/RU2020/000560
(43) Date of publication of application: 27.04.2022
(73) Proprietor: Harman Becker Automotive Systems GmbH, 76307 Karlsbad (DE)
(72) Inventor: FILIMONOV, Andrey Viktorovich, Kamenki 607610 (RU); FILATOVA, Anastasiya Sergeevna, Balakhna 606408 (RU); ALEKSEEVNA, Demareva Valeriya, Nizhny Novgorod 603081 (RU); SHISHALOV, Ivan Sergeevich, Nizhny Novgorod 603074 (RU); SHISHANOV, Sergey Valeryevich, Nizhny Novgorod 603003 (RU); BURASHNIKOV, Evgeny Pavlovich, Nizhny Novgorod 603057 (RU); DEVYATKIN, Anton Sergeevich, Nizhny Novgorod 603142 (RU); SOTNIKOV, Mikhail Sergeevich, Nizhny Novgorod 603003 (RU)
(74) Representative: Rummler, Felix

(56) References cited:
- EP-A1- 2 564 777
- US-A1- 2020 218 935

## Description

### Field

The present disclosure relates to systems and methods for determining eye movements, and more particularly, to determining the eye movements of a driver of a vehicle.

### Background

Eye-tracking is a technique that enables observing and recording eye movements. Recording eyes with a camera and analyzing the images allows determining periods of eye fixation and periods of fast movement between fixations, referred to as saccades. However, the reliability of this technique may be limited in case of bad lighting and high noise, in particular in a vehicle. Therefore, a demand exists for improving the reliability of eye movement determination.

The following publications relate to eye metrics:
- Bulling et al., Proc. 11th Internat. Conf. Ubiquitous Computing, 41. DOI: 10.1145/1620545.1620552 (2009)
- Gibaldi et al., Behav. Res. DOI: 10.3758/s13428-020-01388-2 (2020)
- Lappi et al., Front. Psychol. 8, 620. DOI: 10.3389/fpsyg.2017.00620 (2017)
- WO2006024129A1
- US9475387B2
- US7556377B2
- US7556377B2
- US20160228069A1
- US6102870A
- US20140003658A1

The following publications relate to applications of eye metrics measurement:
- Ma'touq et al., J Med. Eng. Technol. 38, 416. DOI: 10.3109/03091902.2014.968679 (2014)
- Wilkinson et al., JCSM 9, 1315. DOI: 10.5664/jcsm.3278 (2013)
- Jackson et al., Accid. Anal. Prev. 87, 127. DOI: 10.1016/j.aap.2015.11.033 (2016)
- Cori et al., Sleep Med. Rev. 45, 95. DOI: 10.1016/j.smrv.2019.03.004 (2019)
- Alvaro et al. J. Clin. Sleep Med. 12, 1099. DOI: 10.5664/jcsm.6044 (2016)
- Jackson ML et al. Traffic Inj. Prev. 17, 251. DOI: 10.1080/15389588.2015.1055327 (2016)
- François C et al., Int. J. Environ. Res. Public Health 13, 174. DOI: 10.3390/ijerph13020174 (2016)
- Shekari Soleimanloo et al., J. Clin. Sleep Med. 15, 1271. DOI: 10.5664/jcsm.7918 (2019)
- M. Ramzan et al., IEEE Access 7, 61904. DOI: 10.1109/ACCESS.2019.2914373 (2019)
- Arizpe, J. et al., PloS one 7, e31106. DOI: 10.1371/journal.pone.0031106 (2012)
- Rucci, M. et al. Trends Neurosc. 38,195. DOI: 10.1016/j.tins.2015.01.005 (2015)
- Woods et al. Sleep, 36, 1491. DOI: 10.5665/sleep.3042 (2013)
- Alghowinem et al., 2013 IEEE Internat. Conf. Image Proc., 4220, DOI: 10.1109/ICIP.2013.6738869
- Delazer et al., Front. Neurol. 9, 359. DOI: 10.3389/fneur.2018.00359
- Khalife et al. 4th Internat. Conf. Adv. Biomed. Eng. (ICABME), 1, DOI: 10.1109/ICABME.2017.8167534. (2017)

US 2020/218935 A1 describes a reduced attention state estimation system including an eyeball movement and eyelid activity measurement unit that measures an eyeball movement and an eyelid activity of a subject to obtain eyeball movement and eyelid activity data, a section determination unit that determines an eye opening section, an eye closing section, an eye-blinking section and a cluster section based on the eyeball movement and eyelid activity data, an eyeball movement and eyelid activity-related information calculation unit that calculates a sharpness of microsaccade or the like for each eye opening section based on the eyeball movement and eyelid activity data, and an attention assessment unit that determines an attention assessment or the like for an eye opening/cluster section, which is an eye opening section and cluster section, based on the sharpness of microsaccade or the like.

EP 2 564 777 A1 describes a method for improving the reliability of a portion of physiological data from an image sensor monitoring an operator positioned in an operator compartment of a vehicle, the method comprising receiving, from the image sensor, physiological data comprising information relating to at least one of eye, face, head, arms and body motion of the operator, identifying an indication of at least an eyelid closure, eye movement or head movement of the operator based on the physiological data, comparing at least one of the physiological data and a lighting condition within the operator compartment with a predetermined set of rules for a current operator status, and classifying the type of eyelid closure, eye movement and/or head movement by correlating the identified eyelid closure, eye movement and/or head movement and a result of the comparison.

### Summary

The present invention is defined in the independent claims. The dependent claims recite selected optional features.

In the following, each of the described methods, apparatuses, examples, and aspects, which do not fully correspond to the invention as defined in the claims is thus not according to the invention and is, as well as the whole following description, present for illustration purposes only or to highlight specific aspects or features of the claims.

Disclosed and claimed herein are methods and systems for determining eye movement.

A first aspect of the present disclosure relates to a computer-implemented method for determining a movement associated with an eye of a person, the method comprising:
- recording a series of images of the eye;
- based on the recorded images, generating a first signal representative of a movement associated with the eye;
- deriving at least one movement parameter from the first signal,
- for one or more points in time, determining whether the first signal indicates that the eye is a first state, or in a second state, or is inconclusive as to whether the eye is in the first state or the second state, based on whether one or more of the movement parameters exceeds a predetermined threshold; and
- selecting a portion of the first signal based on the determination.

The camera to record the series of images may be a visible light camera or an infrared camera. In addition to the camera, an artificial light source may be used to improve the signal-to-noise ratio. An infrared camera may be used, in particular with an infrared light source, which does not distract the person when in operation. Cameras may optionally be cooled to improve the signal-to-noise ratio. The first signal indicate a maximum distance between the eyelids, a position of the pupil relative to the eyelids, or a pupil diameter. The first signal may be expressed as a time series. The signal may be subject to noise or other spurious effects, such as the person turning the eye away from the camera. Other possible sources of error are lack of mechanical stability of the camera mounting, computational errors in data analysis, and limitations in illumination. These effects may reduce the reliability of the first signal. The movement parameter, as derived from the first signal, may be the signal itself, a linear or non-linear translation of the signal to another frame of reference, a first derivative indicative of a velocity, a second derivative indicative of an acceleration, or the result of more complex calculations. For example a movement parameter may also be the duration of a period of time in which variations in the signal are below a further predetermined threshold. In an embodiment, the movement parameter indicates an amplitude, a duration, a velocity, or a frequency of the movement. The state is determined depending on a comparison of a movement parameter with a predetermined threshold. The threshold may be a physiologically known limit of eye movement, such as a highest possible blink or saccade velocity. The disclosure is thus based on the principle that the reliability of eye movement determination can be increased by taking into account known facts on the physiology of the eye. Although the method is suitable for determining human eye movements, it may alternatively be applied for determining eye movement of certain animals in an embodiment, albeit with different values of the predetermined criteria. Data that contradict the predetermined criteria may be considered inconclusive as to whether the eye is open or closed. The reliability is thereby increased, and there is no need for filters for suppressing noise. Such filters may in general reduce noise, but they also bear the risk of producing different errors. For example, a sliding average filter may reduce outliers, but also flattens a curve to the extent that the signal may incite an erroneous interpretation in subsequent analysis steps. However, the method of the present disclosure may be supplemented by filtering techniques as detailed below.

In a further embodiment,
- the first signal is indicative of eye openness;
- the first state is associated with an open eye;
- the second state is associated with a closed eye; and
- the movement parameters comprise an eye openness and one or more of a blink duration, a number of blinks per minute, a time between blinks, and a maximum blink velocity during a movement of the eyelid when the eye is being opened and/or closed.

Therefore, it is determined whether the first signal indicates an open eye, a closed eye, or is inconclusive as to whether the eye is open or closed. This determination is not only based on a threshold relating to the openness of the eye, as derived, for example, from a signal indicative of the maximum distance between the eyelids. Rather, it is further dependent on predetermined criteria that represent known physiological facts on human eye blinks.

In a further embodiment,
- the first signal is indicative of eye gaze.
- the first state is associated with a saccade;
- the second state is associated with a fixation; and
- the movement parameters comprise an eye movement velocity and one or more of a saccade amplitude, saccade velocity, saccade duration, and fixation duration.

Thereby, based on a signal indicative of a position of a pupil, a saccade may be distinguished from a fixation. Rather than just calculating the eye movement velocity, a second movement parameter is calculated and compared against known physiological parameters, for example the maximum saccade velocity. Alternatively, the signal may have the form of Euler angles for the eyeball position.

The eye movement velocity may be determined by calculating a numeric temporal derivative of the second signal. Performing the calculation for each point in time may refer to calculating a derivative of a curve that represents an angle or a position, wherein each value corresponds to one recorded image of the image series. Alternatively, each value may correspond to an average of a small number of images (e. e. three images) subsequently recorded, in order to reduce noise. The velocity is then compared to a predetermined second threshold to determine whether the eyes move fast, i. e. a saccade is happening, or the eyes move slowly, i. e. the eyes are fixed into one direction.

In a further embodiment, the portion corresponds to one or more periods in time when the eye is open, and the method further comprises:
- based on the recorded images corresponding to the selected portion, generating a second signal indicative of eye gaze;
- deriving at least a third movement parameter indicative of an eye movement velocity from the second signal;
- for one or more points in time, determining whether the second signal indicates a saccade, a fixation, or is inconclusive as to whether the second signal indicates a saccade or a fixation, based on whether one or more of the movement parameters exceeds a predetermined threshold.

Thereby, a saccade or fixation is detected based on a signal indicative of eye gaze, and the analysis is applied only to images for which it has been previously determined with high reliability that the eye is open. In contrast, images that are deemed inconclusive are excluded. Thereby, the method benefits from the increased reliability of the blink detection as detailed above. In a further embodiment, said method further comprises deriving a fourth movement parameter indicative of one or more of a saccade amplitude, saccade velocity, saccade duration, and fixation duration. Thereby, also the saccade or fixation is detected based on physiologically proven criteria.

In a further embodiment, the determination comprises generating an eye movement classification comprising:
- generating a first movement parameter;
- generating a preliminary eye movement classification of the signal as indicative of the first state if the first movement parameter exceeds a first threshold, and as indicative of the second state if the first movement parameter does not exceed the first threshold;
- generating a second movement parameter; and
- for one or more points in time, modifying the preliminary eye movement classification as inconclusive if the second movement parameter exceeds a second threshold, thereby generating the eye movement classification.

This two-step procedure consists in first distinguishing between first and second state, such as between an open and a closed eye, or between a saccade and a fixation, using a first threshold. In a second step, the reliability of the classification is improved by explicitly marking the points in time for which the preliminary classification did not yield physiologically possible results as inconclusive.

In a further embodiment, the method further comprises determining one or more fixation periods, and determining, for each fixation period, an averaged eye gaze signal. The average may be calculated over the duration of the fixation period. This allows identifying a gaze direction. If these steps are performed on data generated by the steps above, the determination of the gaze direction is possible at much higher reliability. In addition, also the accuracy of the determination of the gaze direction is higher. This is because other noise removal steps that may distort the measured data, such as calculating a sliding average, are unnecessary. Furthermore, the beginning and end time of a fixation period may be determined more accurately.

In a further embodiment, the method further comprises filtering measurement noise from the signal. The combination of both filtering noise and using a plurality of movement parameters allows further increasing the reliability.

In a second aspect of the disclosure, a system for determining a cognitive demand level of a user is provided. The system comprises a first computing device, a first sensor, a second computing device, and a second sensor. The system is configured to execute the steps described above. All properties of the computer-implemented method of the present disclosure also apply to the system.

### Brief description of the drawings

The features, objects, and advantages of the present disclosure will become more apparent from the detailed description set forth below when taken in conjunction with the drawings in which like reference numerals refer to similar elements.
**Figure 1** depicts a block diagram of a system according to an embodiment;
**Figure 2** depicts a flow chart representing a method for determining eye openness according to an embodiment; and
**Figure 3** depicts a flow chart representing a method for determining eye movement according to an embodiment.

### Detailed description of the preferred embodiments

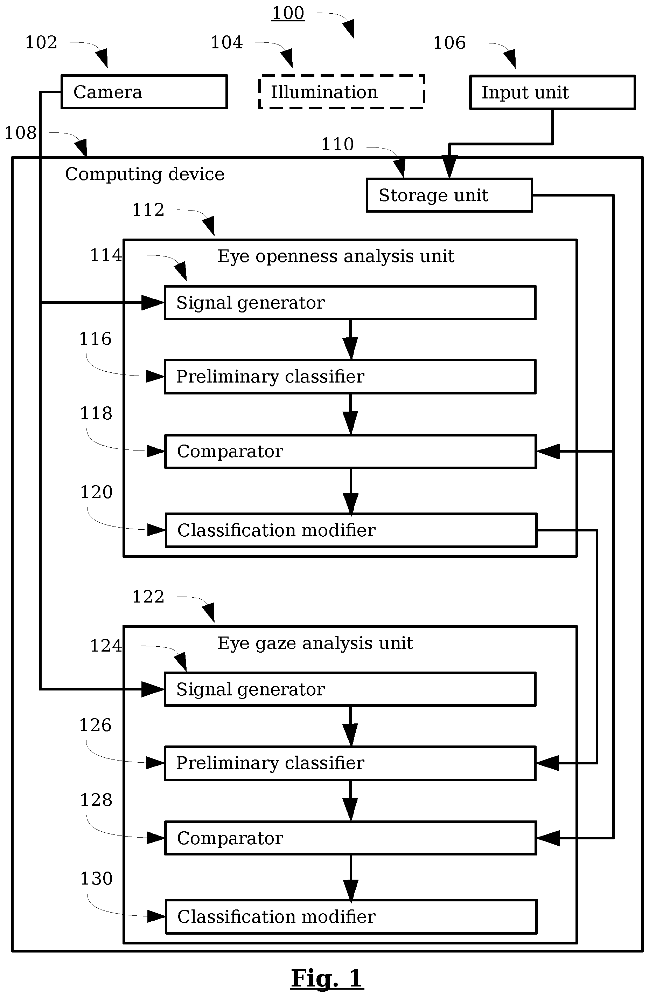

**Figure** 1 depicts a block diagram of a system 100 according to an embodiment. The system comprises a camera 102 to record a series of images of a human eye. An optional illumination unit 104 may provide artificial lighting. The camera may be a visible light or an infrared camera. By using infrared imaging and infrared illumination, the system may be used in the dark, e. g. if the system is attached to or comprised in a vehicle and determining eye movement of a driver at night. However, the present disclosure is not limited to vehicles and may be used in other environments. The input unit 106 is configured to receive one or more predetermined criteria for eye openness and eye movement. The criteria for eye openness may comprise minimum and maximum values for one or more of a blink duration, a number of blinks per minute, a time between blinks, and a maximum blink velocity during a movement of the eyelid when the eye is being opened and/or closed. The eye movement criteria may comprise one or more of a saccade amplitude, saccade velocity, saccade duration, and fixation duration. The criteria may be entered into the system and stored in a storage unit 110, which may comprise a non-volatile memory, in the computing device 108.

Upon operation, the camera 102 records a series of images. The images are first analyzed by an eye openness analysis unit 112, which determines a continuous signal indicative of an eye openness. The signal may relate to a maximum distance between upper and lower eyelid, and it may be subject to noise and measurement errors, such as an erroneous value due to the person turning the head away from the camera. The preliminary classifier 116 determines whether the signal exceeds a first threshold, and classifies the signal at that point in time as indicative of an open eye or a closed eye. Comparator 118 receives the output of the preliminary classifier 116 and determines whether the signal complies with the criteria. If, for example, two blinks are determined at a temporal delay that is below a threshold defined by physiological limits, the system may determine that the signal is not compliant with the criteria. In response to a determination that the signal is not compliant with the criteria, the classification modifier 120 changes the classification in a way that avoids an erroneous signal. For example, the classification may be determined as inconclusive for the period in time in which it is not compliant with the criteria. However, one or more additional or alternative classifications may be generated, that are indicative of an open eye or a closed eye under certain conditions. If, for example, the signal of only one image is indicative of a closed eye, whereas the signal indicates an open eye for the preceding and subsequent images, the signal may be considered indicative of an open eye for the purpose of determination of a blink period.

The image generated by the camera and the output of the eye openness analysis unit 112 are sent into an eye gaze analysis unit 122. The eye gaze analysis unit 122 is configured to analyze the images that have been determined to represent an open eye. Analysis of images depicting closed eyes or images where the first signal is inconclusive is thus avoided, and therefore, a first source of error is mitigated. The corresponding periods in time are marked as inconclusive of the eye gaze direction and movement. For the images representing an open eye, a signal generator 124 generates a signal indicative of the eye gaze, which may be expressed as either a pair of Euler angles, or as positions of the pupil relative to a center of the eye. Furthermore, a numerical derivative is calculated to determine a velocity of the eye movement. The preliminary classifier 126 classifies periods in time where the velocity exceeds a second threshold as saccades, and periods in time where the velocity is below the secondthreshold as fixations. Comparator 128 then verifies for each point in time if the classification complies with the eye movement criteria. If this is not the case, for example if a velocity exceeds a threshold indicative of a physiologically possible eye movement velocity, the classification modifier 130 may set the classification as inconclusive as to whether a fixation or a saccade is present. Thereby, every point in the time during which the images are taken is unambiguously classified as blink, saccade, fixation, or inconclusive.

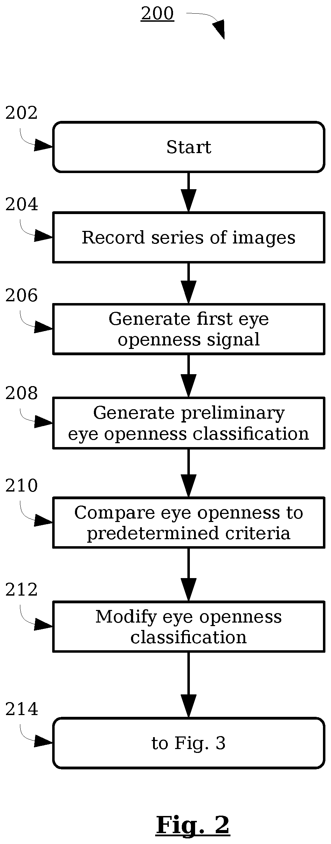

**Figure 2** depicts a flow chart representing a method for determining eye movement according to an embodiment. The method begins, 202, when operation of the system 100 is initiated. If the system 100 is comprised in a vehicle, this may begin, e. g., once the driver is seated in the vehicle. A series of images is recorded, 204. For a practical eye tracker, the camera may run continuously, and the computing device performs a live analysis of the last seconds of video, i. e. the last recorded images. The system may thus analyze a sliding window in time. The images may be subject to noise and measurement errors. If for example, the camera or the head of the person is moving, the eye openness value may be wrong. Other sources of error may comprise electronic noise in case of a low signal in a dark environment, or a saturated camera in case of exposure to sunlight. A first signal of the openness of the eyes, e. g. a maximum distance between upper and lower eyelid, is generated, 206. The signal corresponding to a point in time may either correspond to a single frame or to a plurality of subsequent frames over which the signal is averaged. This defines a smallest time unit to which a point in time refers. Furthermore, filtering techniques as known in the art may be combined with the method of the present disclosure. The eye openness is then preliminarily classified, 208, for example by determining if the signal exceeds a threshold. Thereby, a binary signal is generated. This signal may, however, be subject to errors in case of the aforementioned effects. If, for example, the first signal is fluctuating and close to the threshold, the preliminary classification may create the impression of a fast fluctuation of closed and open eyes, which may physiologically not be possible. The result is then compared, 210, to the predetermined criteria as stored in the data storage unit 110, which may then, for example lead to the determination that the time between blinks as determined is lower than a minimum time between blinks as given in the criteria. In another example, this step may further include determining an experimental blink velocity by calculating a derivative of the first signal and comparing this to a physiological minimum or maximum. For points in time for which the signal does not comply with the criteria (blink duration, blinks per minute, time between blinks, blink velocity), the classification is then modified, 212, to indicate that the signal is inconclusive as to whether the eye is open or closed.

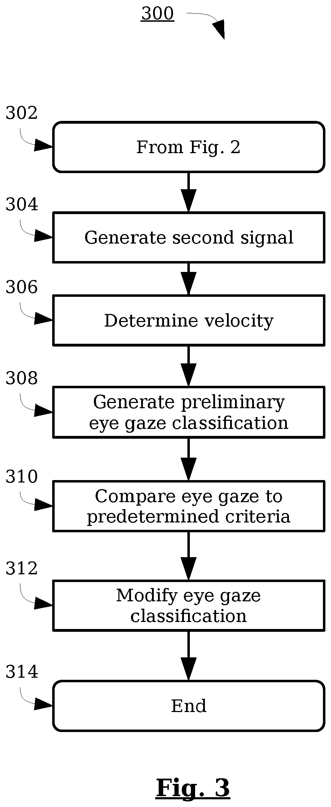

Thereby, the system is prevented from generating an indication of an open or closed eye in case the signal is unclear. Thereby, the reliability of the eye openness classification is increased. The eye openness classification may then be used directly for one or more applications, such as detecting driver drowsiness. Furthermore, the classification may be used for determining eye gaze and/or eye movement features as described with respect to Figure 3.

**Figure 3** depicts a flow chart representing a method 300 for determining eye movement according to an embodiment. The method receives, 202, the camera images and an eye openness classification generated in method 200. A second signal, indicative of eye movement, such as a pair of Euler angles, is generated, 304. The velocity is determined, 306, e. g. by calculating a derivative. A preliminary eye gaze classification is created based on the velocity. Thereby, for each point in time, the velocity is compared to a second threshold, and the data are classified as a saccade in case the velocity exceeds the second threshold, or a fixation in case the velocity is below the second threshold. Hereby, a point in time may refer to a time window of a small size, for example 3 frames, which may be recorded in an interval of 100 milliseconds. The classification is further compared, 310, to the predetermined criteria. For example, the amplitude of the saccade may be determined to a physiologically unrealistic value such as a 100 degrees angle, or a saccade velocity may be determined to be higher than realistic for a human eye. In these cases the corresponding points in time are reclassified as inconclusive. Furthermore, a check is performed if the saccades and fixations form contiguous periods in time that comply with predetermined criteria for minimum and maximum durations of saccades and fixations. Periods in time that do not comply with the criteria are reclassified as inconclusive, 312. Thereby, the reliability of the eye movement determination method is increased. Furthermore, filtering the first and second signals is optionally possible, but it is not necessary. Thereby, possible distortion of the signal is avoided, and all features of the signal are preserved.

In the end, 314, the classification of the eye gaze is provided for further analysis. For example, for the fixation periods, an averaged direction may be determined. Thereby, it can be determined at which object the person is looking. This information may be used for an augmented reality system. The data may also be used for determining a cognitive demand.

### Reference signs

- 100: System
- 102: Camera
- 104: Illumination
- 106: Input unit
- 108: Computing device
- 110: Storage unit
- 112: Eye openness analysis unit
- 114: Signal generator
- 116: Preliminary classifier
- 118: Comparator
- 120: Classification modifier
- 122: Eye gaze analysis unit
- 124: Signal generator
- 126: Preliminary classifier
- 128: Comparator
- 130: Classification modifier
- 200: Method for determining eye openness
- 202-214: Steps of method 200
- 300: Method for determining eye movement
- 302-314: Steps of method 300

## Claims

1. Computer-implemented method (200) for determining a movement associated with an eye of a person, the method comprising:
recording (204) a series of images of the eye;
based on the recorded images, generating (206) a first signal representative of a movement associated with the eye;
for one or more points in time, determining whether the first signal indicates that the eye is in a first state, or in a second state, or is inconclusive as to whether the eye is in the first state or the second state;
wherein the determination comprises generating an eye movement classification comprising:
generating a first movement parameter from the first signal;
generating a preliminary eye movement classification of the first signal as indicative of the first state if the first movement parameter exceeds a predetermined first threshold, and as indicative of the second state if the first movement parameter does not exceed the first threshold;
generating a second movement parameter from the first signal; and
modifying the preliminary eye movement classification as inconclusive if the second movement parameter exceeds a second threshold, thereby generating the eye movement classification;
wherein the second threshold indicates a physiological limit of eye movement; and
selecting a portion of the first signal excluding images that are classified inconclusive;
wherein the first signal is indicative of eye openness, the first state is associated with an open eye, and the second state is associated with a closed eye; or
wherein the first signal is indicative of eye gaze, the first state is associated with a saccade, and the second state is associated with a fixation.

2. The method (200) of claim 1,
wherein the first and second movement parameters indicate an amplitude, a duration, a velocity, or a frequency of the movement.

3. The method (200) of claim 1 or 2,
wherein the first signal is indicative of eye openness,
wherein the first state is associated with an open eye;
wherein the second state is associated with a closed eye; and
wherein the first and second movement parameters comprise
an eye openness and
one or more of a blink duration, a number of blinks per minute, a time between blinks, and a maximum blink velocity during a movement of the eyelid when the eye is being opened and/or closed.

4. The method (200) of claim 1 or 2,
wherein the first signal is indicative of eye gaze,
wherein the first state is associated with a saccade;
wherein the second state is associated with a fixation; and
wherein the first and second movement parameters comprise
an eye movement velocity and
one or more of a saccade amplitude, saccade velocity, saccade duration, and fixation duration.

5. The method (200) of claim 3,
wherein the portion corresponds to one or more periods in time when the eye is open, and the method further comprises:
based on the recorded images corresponding to the selected portion, generating a second signal indicative of eye gaze;
deriving a third movement parameter indicative of an eye movement velocity from the second signal;
for one or more points in time, generating a further preliminary classification by determining whether the second signal indicates a saccade or a fixation, based on whether the third movement parameter exceeds a predetermined third threshold.

6. The method of claim 5, further comprising:
deriving a fourth movement parameter indicative of one or more of a saccade amplitude, saccade velocity, saccade duration, and fixation duration; and
modifying the further preliminary classification as inconclusive based on whether the fourth movement parameter exceeds a fourth threshold.

7. The method (200) of any of claims 4-6, further comprising:
determining one or more fixation periods, and
determining, for each fixation period, an averaged eye gaze signal.

8. The method (200) of any preceding claim, further comprising filtering measurement noise from the signal.

9. A system (100) for determining an eye movement associated with an eye of a person, the system comprising:
a camera (102) configured to record a series of images of the eye; and
a computing device (108) configured to execute the method of any of the preceding claims.

## Patentansprüche

1. Computerimplementiertes Verfahren (200) zum Bestimmen einer Bewegung, die einem Auge einer Person zugeordnet ist, wobei das Verfahren Folgendes umfasst:
Aufzeichnen (204) einer Bilderserie des Auges;
basierend auf den aufgezeichneten Bildern Erzeugen (206) eines ersten Signals, das eine dem Auge zugeordnete Bewegung darstellt;
für einen oder mehrere Zeitpunkte Bestimmen, ob das erste Signal angibt, dass sich das Auge in einem ersten Zustand oder in einem zweiten Zustand befindet, oder ob es nicht eindeutig ist, ob sich das Auge in dem ersten Zustand oder in dem zweiten Zustand befindet;
wobei das Bestimmen das Erzeugen einer Augenbewegungsklassifizierung umfasst, die Folgendes umfasst:
Erzeugen eines ersten Bewegungsparameters aus dem ersten Signal;
Erzeugen einer vorläufigen Augenbewegungsklassifizierung des ersten Signals als Angabe zum ersten Zustand, wenn der erste Bewegungsparameter einen vorgegebenen ersten Schwellenwert überschreitet, und als Angabe für den zweiten Zustand, wenn der erste Bewegungsparameter den ersten Schwellenwert nicht überschreitet;
Erzeugen eines zweiten Bewegungsparameters aus dem ersten Signal; und
Modifizieren der vorläufigen Augenbewegungsklassifizierung als nicht eindeutig, wenn der zweite Bewegungsparameter einen zweiten Schwellenwert überschreitet, wodurch die Augenbewegungsklassifizierung erzeugt wird;
wobei der zweite Schwellenwert eine physiologische Grenze der Augenbewegung angibt; und Auswählen eines Abschnitts des ersten Signals unter Ausschluss von Bildern, die als nicht eindeutig klassifiziert werden;
wobei das erste Signal Augenöffnung angibt, dem ersten Zustand ein offenes Auge zugeordnet ist und dem zweiten Zustand ein geschlossenes Auge zugeordnet ist; oder
wobei das erste Signal Blickrichtung angibt, dem ersten Zustand eine Sakkade zugeordnet ist und dem zweiten Zustand eine Fixation zugeordnet ist.

2. Verfahren (200) nach Anspruch 1,
wobei der erste und der zweite Bewegungsparameter eine Amplitude, eine Dauer, eine Geschwindigkeit oder eine Frequenz der Bewegung angeben.

3. Verfahren (200) nach Anspruch 1 oder 2,
wobei das erste Signal Augenöffnung angibt,
wobei dem ersten Zustand ein offenes Auge zugeordnet ist;
wobei dem zweiten Zustand ein geschlossenes Auge zugeordnet ist; und
wobei der erste und der zweite Bewegungsparameter eine Augenöffnung und
eines oder mehrere von einer Blinzeldauer, einer Anzahl von Blinzeln pro Minute, einer Zeit zwischen Blinzeln und einer maximalen Blinzelgeschwindigkeit während einer Bewegung des Augenlids beim Öffnen und/oder Schließen des Auges umfassen.

4. Verfahren (200) nach Anspruch 1 oder 2,
wobei das erste Signal Blickrichtung angibt,
wobei dem ersten Zustand eine Sakkade zugeordnet ist;
wobei dem zweiten Zustand eine Fixation zugeordnet ist; und
wobei der erste und der zweite Bewegungsparameter eine Augenbewegungsgeschwindigkeit und
eines oder mehrere von einer Sakkadenamplitude, Sakkadengeschwindigkeit, Sakkadendauer und Fixationsdauer umfassen.

5. Verfahren (200) nach Anspruch 3,
wobei der Abschnitt einem oder mehreren Zeiträumen entspricht, in denen das Auge geöffnet ist, und das Verfahren ferner Folgendes umfasst:
basierend auf den aufgezeichneten Bildern, die dem ausgewählten Abschnitt entsprechen, Erzeugen eines zweiten Signals, das die Blickrichtung angibt;
Ableiten eines dritten Bewegungsparameters, der eine Augenbewegungsgeschwindigkeit aus dem zweiten Signal angibt;
für einen oder mehrere Zeitpunkte Erzeugen einer weiteren vorläufigen Klassifizierung durch Bestimmen, ob das zweite Signal eine Sakkade oder eine Fixation angibt, basierend darauf, ob der dritte Bewegungsparameter einen vorgegebenen dritten Schwellenwert überschreitet.

6. Verfahren nach Anspruch 5, ferner umfassend:
Ableiten eines vierten Bewegungsparameters, der eines oder mehrere von einer Sakkadenamplitude, Sakkadengeschwindigkeit, Sakkadendauer und Fixationsdauer angibt, und
Modifizieren der weiteren vorläufigen Klassifizierung als nicht eindeutig, basierend darauf, ob der vierte Bewegungsparameter einen vierten Schwellenwert überschreitet.

7. Verfahren (200) nach den Ansprüchen 4 bis 6, ferner umfassend:
Bestimmen einer oder mehrerer Fixationsperioden und
Bestimmen eines gemittelten Blickrichtungssignals für jede Fixationsperiode.

8. Verfahren (200) nach einem der vorhergehenden Ansprüche, ferner umfassend Herausfiltern von Messrauschen aus dem Signal.

9. System (100) zum Bestimmen einer Augenbewegung, die einem Auge einer Person zugeordnet ist, wobei das System Folgendes umfasst:
eine Kamera (102), die konfiguriert ist, um eine Bilderserie des Auges aufzuzeichnen; und
eine Rechenvorrichtung (108), die konfiguriert ist, um das Verfahren nach einem der vorhergehenden Ansprüche auszuführen.

## Revendications

1. Procédé mis en œuvre par ordinateur (200) pour déterminer un mouvement associé à l'œil d'une personne, le procédé comprenant :
l'enregistrement (204) d'une série d'images de l'œil ;
sur la base des images enregistrées, la génération (206) d'un premier signal représentatif d'un mouvement associé à l'œil ;
pour un ou plusieurs moments, le fait de déterminer si le premier signal indique que l'œil est dans un premier état, ou dans un second état, ou non concluant s'il n'est pas possible de déterminer si l'œil est dans le premier état ou le second état ;
dans lequel la détermination comprend la génération d'une classification de mouvement oculaire comprenant :
la génération d'un premier paramètre de mouvement à partir du premier signal ;
la génération d'une classification préliminaire de mouvement oculaire du premier signal comme indiquant le premier état si le premier paramètre de mouvement dépasse un premier seuil prédéterminé, et comme indiquant le second état si le premier paramètre de mouvement ne dépasse pas le premier seuil ;
la génération d'un deuxième paramètre de mouvement à partir du premier signal ; et
la modification de la classification préliminaire de mouvement oculaire comme non concluante si le deuxième paramètre de mouvement dépasse un deuxième seuil, générant ainsi la classification de mouvement oculaire ;
dans lequel le deuxième seuil indique une limite physiologique du mouvement oculaire ; et la sélection d'une partie du premier signal à l'exclusion des images classées comme non concluantes ;
dans lequel le premier signal indique l'ouverture des yeux, le premier état est associé à un œil ouvert et le second état est associé à un œil fermé ; ou
dans lequel le premier signal indique le regard, le premier état est associé à une saccade et le second état est associé à une fixation.

2. Procédé (200) selon la revendication 1,
dans lequel les premier et deuxième paramètres de mouvement indiquent une amplitude, une durée, une vitesse ou une fréquence du mouvement.

3. Procédé (200) selon la revendication 1 ou 2,
dans lequel le premier signal indique l'ouverture des yeux,
dans lequel le premier état est associé à un œil ouvert ;
dans lequel le second état est associé à un œil fermé ; et
dans lequel les premier et deuxième paramètres de mouvement comprennent
une ouverture des yeux et
l'un ou plusieurs des éléments suivants : la durée de clignement, le nombre de clignements par minute, le temps entre les clignements et la vitesse maximale de clignement lors d'un mouvement de la paupière lorsque l'œil est ouvert et/ou fermé.

4. Procédé (200) selon la revendication 1 ou 2,
dans lequel le premier signal indique le regard,
dans lequel le premier état est associé à une saccade ;
dans lequel le second état est associé à une fixation ; et
dans lequel les premier et deuxième paramètres de mouvement comprennent
une vitesse de mouvement oculaire et
l'un ou plusieurs des éléments suivants : amplitude de saccade, vitesse de saccade, durée de saccade et durée de fixation.

5. Procédé (200) selon la revendication 3,
dans lequel la partie correspond à une ou plusieurs périodes pendant lesquelles l'œil est ouvert, et le procédé comprend en outre :
sur la base des images enregistrées correspondant à la partie sélectionnée, la génération d'un second signal indiquant le regard ;
la dérivation d'un troisième paramètre de mouvement indiquant la vitesse de mouvement oculaire provenant du second signal ;
pour un ou plusieurs moments, la génération d'une classification préliminaire supplémentaire en déterminant si le second signal indique une saccade ou une fixation, sur la base du fait que le troisième paramètre de mouvement dépasse ou non un troisième seuil prédéterminé.

6. Procédé selon la revendication 5, comprenant en outre :
la dérivation d'un quatrième paramètre de mouvement indiquant l'un ou plusieurs des éléments suivants : amplitude de saccade, vitesse de saccade, durée de saccade et durée de fixation ; et
la modification de la classification préliminaire supplémentaire comme non concluante sur la base du fait que le quatrième paramètre de mouvement dépasse ou non un quatrième seuil.

7. Procédé (200) selon l'une quelconque des revendications 4 **à 6,** comprenant en outre :
la détermination d'une ou plusieurs périodes de fixation, et
la détermination, pour chaque période de fixation, d'un signal de regard moyen.

8. Procédé (200) selon une quelconque revendication précédente, comprenant en outre le filtrage du bruit de mesure provenant du signal.

9. Système (100) pour déterminer un mouvement oculaire associé à l'œil d'une personne, le système comprenant :
une caméra (102) configurée pour enregistrer une série d'images de l'œil ; et
un dispositif informatique (108) configuré pour exécuter le procédé selon l'une quelconque des revendications précédentes.
